Europäisches Patentamt

European Patent Office

Office européen des brevets

(19)

(11) Veröffentlichungsnummer: **0 367 979**

**A1**

(12) # EUROPÄISCHE PATENTANMELDUNG

(21) Anmeldenummer: **89118255.2**

(22) Anmeldetag: **02.10.89**

(51) Int. Cl.5: **C08K 5/11, C08L 21/00, C07C 69/34**

Patentanspruch für folgenden Vertragsstaat: ES.

(30) Priorität: **06.10.88 JP 250801/88**

(43) Veröffentlichungstag der Anmeldung:
**16.05.90 Patentblatt 90/20**

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Yamamura, Yoshitami**
**64-1, Yasuda Onoe-cho**
**Kakogawa-shi Hyogo-ken(JP)**
Erfinder: **Ogawa, Kiyoshi**
**105-2, Ikenouchi**
**Aioi-shi Hyogo-ken(JP)**
Erfinder: **Miyaura, Tadao**
**2-16-25-1304, Seiiku Joto-ku**
**Osaka-shi Osaka-fu(JP)**
Erfinder: **Ishikawa, Hitoshi**
**4-1, Nakaochiai Suma-ku**
**Kobe-shi Hyogo-ken(JP)**

(54) **Compoundiertes Kautschukgemisch.**

(57) Bei einem compoundierten Kautschukgemisch sollten die Tieftemperatureigenschaften verbessert werden. Dies gelang dadurch, daß als Weichmacher ein synthetisches Esterprodukt eingesetzt wurde, das durch Veresterung einer oligomerisierten Fettsäure mit einem verzweigten primäre 8 bis 32 C-Atome aufweisenden Alkohol eingesetzt wurde.

EP 0 367 979 A1

## Compoundiertes Kautschukgemisch

Die vorliegende Erfindung bezieht sich auf eine neue Art von Kautschukweichmacher - konkret ausgedrückt, einen synthetischen Ester aus einer polymerisierten Fettsäure und mit einer verzweigten Kette im Molekül; dieser Ester soll nach Beimengung in einem compoundierten Kautschukgemisch seine Weichheit in kalter Atmosphäre verbessern und folglich ein stabileres Fahrverhalten auf einer verschneiten/vereisten Straße sicherstellen.

Um die Stabilität des Fahrverhaltens - d.h. die Sicherheit - eines Personenwagens auf einer verschneiten/vereisten Straße zu verbessern, wurde in den letzten Jahren ein Verfahren untersucht, bei dem Spikes in die Reifenlauffläche getrieben wurden, wodurch eine Verbesserung der Stabilität des Fahrverhaltens auf vereisten Straßen erreicht wurde. Allerdings verursacht das Fahren eines Wagens mit Reifen, deren Lauffläche mit Spikes bestückt ist, auf der Straße in einer Jahreszeit, in der Schnee und Eis bereits im Weg- oder Abtauen begriffen sind, zwangsläufig einen Abrieb der asphaltierten Straßenoberfläche. Dies hat zur Folge, daß nicht nur der Straßenbelag beschädigt wird, sondern daß auch infolge der sich abriebbedingt bildenden Asphaltflugstäube eine Luftverschmutzung entsteht, und das ist ein ernstes Problem für die menschliche Umwelt.

Daher besteht nach wie vor ein nachhaltiger Bedarf an Autoreifen ohne Spikes-Bestückung der Reifen, damit man einen Wagen auf einer verschneiten/vereisten Straße stabil und sicher fahren kann. Zu diesem Zweck bestand bekanntlich die Idee, für die Lauffläche des Reifens einen Kautschuk zu verwenden, der eine genügende Weichheit bei kalter Außenluft aufweist (vgl. Kokai-Sho 61-66733, Kokai-Sho 59-206208, Kokai-Sho 59-206209, Kokai-Sho 60-215403), indem man dem Kautschukrohmaterial einen esterartigen Niedertemperaturweichmacher und/oder einen naphten- oder paraffinartigen Weichmacher beimischt. Als konkrete Beispiele für diese Niedertemperaturweichmacher sind in der Literatur esterartige Weichmacher wie beispielsweise phthalsäureartige Diester, repräsentativ sei hier Dioktylphthalsäureester (DOP) usw. genannt, fettsäureartige einbasische Säureester u.a.m. genannt worden; diese esterartigen Niedertemperaturweichmacher und/oder naphthen- oder paraffinartigen Weichmacher besitzen nachweislich die Fähigkeit, dem Kautschuk in kalter Atmosphäre eine verbesserte Weichheit zu verleihen; allerdings erfüllt ihre Weichmacherwirkung bei niedrigen Temperaturen bei weitem noch nicht die Erwartungen, wenn man außerdem zuviel von ihnen beimischt, um die Weichmacherwirkung weiter zu verbessern, entsteht das Problem, daß sie an der Kautschukoberfläche ausbluten. Andererseits führt wegen des relativ niedrigen Molekulargewichts die Sublimierung des Weichmacherbestandteils manchmal im Laufe der Zeit zu einem allmählichen Nachlassen der Weichheit.

Aufgabe der vorliegenden Erfindung ist es daher in Anbetracht der verschiedenen vorgenannten Probleme bei den herkömmlichen Verfahren, ein compoundiertes Kautschukgemisch vorzuschlagen, das eine ausreichende Weichheit bei niedriger Lufttemperatur aufweist, damit man auf einer verschneiten/vereisten Straße stabil und sicher autofahren kann, ohne deswegen (die Lauffläche der Reifen) mit Spikes bestücken zu müssen.

Gegenstand der Erfindung ist ein compoundiertes Kautschukgemisch, dadurch gekennzeichnet, daß es als Weichmacher ein synthetisches Esterprodukt enthält, das durch Veresterung einer oligomerisierten Fettsäure mit einem verzweigten primären, 8 bis 32 C-Atome aufweisenden Alkohol erhalten werden kann.

Ein weiterer Gegenstand der Erfindung sind die Ester einer durch Di-und/oder Trimerisierung von ungesättigten Fettsäuren hergestellten mehrfunktionellen Carbonsäure mit durch Guerbet-Reaktion gewonnenen 2-Alkylalkanolen mit 8 bis 32 C-Atomen.

Nach der vorliegenden Erfindung kann man das folgende Ziel dadurch erreichen, daß man dem compoundierten Kautschukgemisch ein synthetisches Esterprodukt zumischt, das durch Kondensation einer oligomerisierten Fettsäure mit einem verzweigten primären Alkohol hergestellt worden ist. Geeignet sind hier Kondensationsprodukte oligomerisierter Fettsäuren, beispielsweise oligomerisierter $C_{18}$ oder $C_{16}$-Fettsäuren mit verzweigten primären Alkoholen, die 8 bis 32 C-Atome besitzen. Unter den verzweigten primären Alkoholen sind die durch GUERBET-Reaktion von primären unverzweigten Alkoholen zugänglichen 2-Alkylalkanole bevorzugt.

Dies hat den Vorteil, daß der betreffende Ester wegen seines hohen Molekulargewichts eine geringere Sublimierungsneigung aufweist und -was noch günstiger ist - überdies den Vorteil besitzt, daß die Weichheit eines compondierten Kautschukgemisches mit einem solchen synthetischen Ester als Weichmacher bei niedriger Lufttemperatur andere Niedertemperaturplastizitätswerte erheblich übersteigt.

Beispiele für die oligomerisierten Fettsäuren nach der vorliegenden Erfindung sind allgemein als Dimersäure usw. bekannt, ferner als Trimersäure u.a.: Man erhält sie durch Destillierungsreinigung des mit Hilfe von Aktivton als Katalysator bei über 200°C polymerisierten Produkts einer Pflanzenölfettsäure, die ungesättigte Fettsäuren wie Ölsäure, Linolsäure, Linolensäure usw. enthält, zum Beispiel Tallölfettsäure, Sojabohnenfettsäure, Reiskleiefettsäure usw. Eine gehärtete Dimersäure kann ebenfalls benutzt werden.

Der obenrwähnte verzweigte primäre Alkohol mit 8 bis 20 C-Atomen kann in 2-Ethylhexylalkohol und anderen verzweigten Alkoholen mit über 8 C-Atomen bestehen, beispielsweise kann ein solcher verzweigter primärer Alkohol durch die bei 250 - 300° C durch eine Guerbet-Reaktion erfolgende Kondensation eines niedermolekularen Alkohols hergestelllt werden. Solche Alkohole bezeichnet man im allgemeinen als Guerbet-Alkohole mit einer Anzahl von derzeit bis zu 32 C-Atomen.

In den erfindungsgemäßen synthetischen Esterprodukten sind vorzugsweise alle oder fast alle Säuregruppen mit einem Alkohol verestert. Der Bereich des Gehalts an synthetischem Ester nach der vorliegenden Erfindung liegt vorzugsweise bei 5 bis 45 Gewichtsteilen auf 100 Gewichtsteile Kautschuk.

Für diese Erfindung in Frage kommende Kautschukarten sind vorzugsweise Mischkautschuksorten, die aus einem Butadienkautschuk und Styrol-Butadien-Copolymerkautschuk bestehen.

Der synthetische Ester nach der vorliegenden Erfindung wird einem für die Reifenlauffläche benutzten, compoundierten Kautschukgemisch beigegeben, um die Weichheit bei kalter Außenluft zu erreichen. Er kann allerdings auch für andere Kautschukprodukte eingesetzt werden, bei denen eine ähnliche Weichheit erforderlich ist; in diesem Fall kann die Menge der Beimischung nach den Solleigenschaften des betreffenden (End)Produkts eingestellt werden.

Einem compoundierten Kautschukgemisch nach der vorliegenden Erfindung kann jedes allgemein übliche Kautschukmischungsadditiv in entsprechender Menge zugegeben werden, wie beispielsweise Weichmacheröle, Plastifiziermittel, Füllstoffe, Vulkanisiermittel, Vulkanisationsbeschleuniger, Klebrigmacher, Formenlösestoffe, Pigmente, Alterungsschutzmittel, Metallseifen, Verarbeitungshilfsmittel usw.; die gleichzeitige Beimischung eines herkömmlichen Weichmachers zusammen mit dem synthetischen Ester nach der vorliegenden Erfindung ist ebenfalls problemlos möglich.

## Beispiele

Nachstehend wird die vorliegende Erfindung anhand von Beispielen näher beschrieben. Die Vermischung des Kautschuks in Beispiel 1 - 5 und dem Vergleichsbeispiel erfolgte unter Benutzung einer offenen Rolle von 10 x 16" mit einer Rollenumdrehung von 19 : 24 U/min bei einer Rollentemperatur zwischen Raumtemperatur und 50° C - alles nach dem herkömmlichen Verfahren.

Die Rezepturen der wie beschrieben verarbeiteten compoundierten Kautschukgemische von Beispiel 1 - 5 sowie die Härtewerte nach JIS A bei Lufttemperaturen zwischen 20 und -40° C werden in Tabelle 1 gezeigt.

Aus dieser Tabelle 1 ist ersichtlich, daß die compoundierten Kautschukgemische nach der vorliegenden Erfindung mit sinkender Temperatur im Vergleich zu dem herkömmlichen Kautschukgemisch nicht spürbar härter werden, außerdem ist Schwankungsbreite der temperaturabhängigen Härte bei den erstgenannten Gemischen kleiner als bei letzterem. Folglich können die compoundierten Kautschukgemische nach der vorliegenden Erfindung auf einer verschneiten/vereisten Straße hervorragend benutzt werden.

Tabelle 1

| | Beispiele | | | | | |
|---|---|---|---|---|---|---|
| | Vergl. 1 | 1 | 2 | 3 | 4 | 5 |
| SBR-Kautschuk | 70 | 70 | 70 | 70 | 70 | 70 |
| BR-Kautschuk | 30 | 30 | 30 | 30 | 30 | 30 |
| Stearinsäure | 2 | 2 | 2 | 2 | 2 | 2 |
| Zinkoxid Nr. 1 | 3 | 3 | 3 | 3 | 3 | 3 |
| Ruß (HAF) | 85 | 85 | 85 | 85 | 85 | 85 |
| Aromatisches Weichmacheröl | 42 | | | | | |
| Probe A | | 42 | | | | |
| Probe B | | | 42 | | | |
| Probe C | | | | 42 | | |
| Probe D | | | | | 42 | |
| Probe E | | | | | | 42 |
| Alterungsschutzmittel (IPPD) | 1 | 1 | 1 | 1 | 1 | 1 |
| Vulkanisationsbeschleuniger (OBS) | 1,2 | 1,2 | 1,2 | 1,2 | 1,2 | 1,2 |
| Schwefel | 1,5 | 1,5 | 1,5 | 1,5 | 1,5 | 1,5 |
| GESAMT | 235,7 | 235,7 | 235,7 | 235,7 | 235,7 | 235,7 |
| Kautschukgehalt (%) | 42,4 | 42,4 | 42,4 | 42,4 | 42,4 | 42,4 |
| Härte n. JIS A bei 20 °C | 52 | 44 | 45 | 46 | 45 | 50 |
| 0 °C | 56 | 44 | 48 | 49 | 47 | 50 |
| -20 °C | 70 | 56 | 56 | 58 | 58 | 60 |
| -40 °C | 89 | 71 | 71 | 73 | 73 | 75 |

Probe A - E in Tabelle 1 wurde jeweils folgendermaßen synthetisiert:

Probe A:

Zuerst werden 560g (1 mol) Dimersäure (Versadyme 288), hergestellt von Henkel Corp., und 260 g (2,0 Mol) 2-Ethylhexylalkohol zusammen vermischt, danach werden 0,82 g (0,1 Gew.-%) p-Toluolsulfonsäure zugegeben, dann wird die gesamte Mischung einer Dehydrierung/Kondensation in gasförmiger Stickstoffatmosphäre bei Erhitzung auf 180 °C für 4 Stunden unter Umrühren unterzogen, um damit Probe A zu erhalten. Die so erhaltene Probe A ist gekennzeichnet durch die Säurezahl 2,8, die Verseifungszahl 143 und die Viskosität von 138 cSt/40 °C.

Probe B:

Zuerst werden 560 g (1,0 mol) gehärtete Dimersäure (Versadyme 52, hergestellt von Henkel Corp.) und 260 g (2,0 mol) 2-Ethylenhexylalkohol zusammen vermischt, danach werden 0,82 g (0,1 Gew.-%) p-Toluolsulfonsäure zugegeben, dann wird die gesamte Mischung einer Kondensation ähnlich wie bei Probe A unterzogen, um somit Probe B zu erhalten. Die so erhaltene Probe B ist gekennzeichnet durch die Säurezahl 1,6, die Verseifungszahl 144 und die Viskosität von 132 cSt/40 °C.

Probe C:

Zuerst werden 840 g (1,0 mol) Trimersäure (Versatryme 213, hergestellt von Henkel Corp.) und 390 g (3,0 mol) 2-Ethylenhexylakohol zusammen vermischt, danach werden 1,2 g (0,1 Gew.-% p-Toluolsulfonsäure zugegeben, dann wird die gesamte Mischung einer Kondensation ähnlich wie bei Probe A unterzogen,

4

um somit Probe C zu erhalten. Die so erhaltene Probe C ist gekennzeichnet durch die Säurezahl 4,0, die Verseifungszahl 139 und die Viskosität von 168 cSt/40°C.

Probe D:

Zuerst werden 560 g (1,0 mol) Versadyme 288 und 596 g (2 mol) Rilanit 620 (hergestellt von Henkel KGaA) zusammen vermischt, danach werden 1,2 g (0,1 Gew.-% p-Toluolsulfonsäure zugegeben, dann wird die gesamte Mischung einer Dehydrierung/Kondensation in gasförmiger Stickstoffatmosphäre bei Erhitzung auf 220°C für 4 Stunden unter Umrühren unterzogen, um somit Probe D zu erhalten. Die so erhaltene Probe D ist gekennzeichnet durch die Säurezahl 3,5, die Verseifungszahl 98 und die Viskosität von 147 cSt/40°C.

Probe E:

Zuerst werden 840 g (1 mol) Versatryme 213 und 894 g (3,0 mol) Rilanit G20 zusammen vermischt, danach werden 1,7 g (0,1 Gew.-%) p-Toluolsulfonsäure zugegeben, dann wird die gesamte Mischung einer Kondensation ähnlich wie bei Probe D unterzogen, um somit Probe E zu erhalten. Die so erhaltene Probe E ist gekennzeichnet durch die Säurezahl 4,8, die Verseifungszahl 103 und die Viskosität von 295 cSt/40°C.
SBR: Styrol-Butadien-Copolymerkautschuk
(Nippon Synthetic Rubber Co., Ltd., Nr. 1500)
BR: Butadien-Kautschuk
(Nippon Synthetic Rubber Co., Ltd., Nr. BR01)
IPPD: N-Phenyl-N'-Isopropyl-P-Phenylendiamin
OBS: N-Oxydiethylen-2-Benzothiazylsulfenamid
Aromatisches Weichmacheröl
(Nippon Synthetic Rubber Co., Ltd., Nr. JSR Aroma)
Rilanit G20: Guerbet-Alkohol mit 20 C-Atomen (hergestellt von Henkel KGaA)

**Ansprüche**

1. Ein compondiertes Kautschukgemisch, dadurch gekennzeichnet, daß es als Weichmacher ein synthetisches Esterprodukt enthält, das durch Veresterung einer oligomerisierten Fettsäure mit einem verzweigten primären, 8 bis 32 C-Atome aufweisenden Alkohol erhalten werden kann.

2. Kautschukgemisch nach Anspruch 1, dadurch gekennzeichnet, daß als Weichmacher das Umsetzungsprodukt einer oligomerisierten Fettsäuremischung auf Basis von $C_{16}$ oder $C_{18}$ Fettsäuren eingesetzt wird.

3. Kautschukgemisch nach den Ansprüchen 1 und 2, dadurch gekennzeichnet, daß als Weichmacher ein Veresterungsprodukt einer oligomerisierten Fettsäure mit einem durch GUERBET-Reaktion gewonnenen primären 2-Alkylalkanol eingesetzt wird.

4. Kautschukgemisch nach den Ansprüchen 1 bis 3, dadurch gekennzeichnet, daß das als Weichmacher eingesetzte synthetische Esterprodukt vorzugsweise 32 bis 40 C-Atome im Säureteil und vorzugsweise 16 bis 20 C-Atome im Alkoholteil enthält.

5. Kautschukgemisch nach den Ansprüchen 1 bis 4, dadurch gekennzeichnet, daß das synthetische Esterprodukt in Mengen von 5 bis 45 Gew.-Teilen, bezogen auf 100 Gewichtsteile Kautschuk, eingesetzt wird.

6. Ester einer durch Di- und/oder Trimerisierung von ungesättigten Fettsäuren hergestellten mehrfunktionellen Carbonsäure mit durch Guerbet-Reaktion gewonnenen 2-Alkylalkanolen mit 8 bis 32 C-Atomen.

Patentansprüche für folgenden Vertragsstaat: ES

1. Verfahren zur Herstellung eines compondierten Kautschukgemisches, dadurch gekennzeichnet, daß man eine oligomerisierte Fettsäure mit einem verzweigten primären, 8 bis 32 C-Atome enthaltenden Alkohol verestert und das so erhaltene synthetische Esterprodukt einem Kautschukgemisch als Weichmacher zusetzt.

2. Verfahren nach Anspruch 1, dadurch gekennzeichnet, daß als Weichmacher das Umsetzungsprodukt einer oligomerisierten Fettsäuremischung auf Basis von $C_{16}$ oder $C_{18}$ Fettsäuren eingesetzt wird.

3. Verfahren nach den Ansprüchen 1 und 2, dadurch gekennzeichnet, daß als Weichmacher ein Veresterungsprodukt einer oligomerisierten Fettsäure mit einem durch GUERBET-Reaktion gewonnenen primären 2-Alkylalkanol eingesetzt wird.

4. Verfahren nach den Ansprüchen 1 bis 3, dadurch gekennzeichnet, daß das als Weichmacher eingesetzte synthetische Esterprodukt vorzugsweise 32 bis 40 C-Atome im Säureteil und vorzugsweise 16 bis 20 C-Atome im Alkoholteil enthält.

5. Verfahren nach den Ansprüchen 1 bis 4, dadurch gekennzeichnet, daß das synthetische Esterprodukt in Mengen von 5 bis 45 Gew.-Teilen, bezogen auf 100 Gewichtsteile Kautschuk, eingesetzt wird.

6. Verfahren zur Herstellung von Estern, dadurch gekennzeichnet, daß man die Di- und/oder Trimerisierungsprodukte ungesättigter Fettsäuren mit durch Guerbet-Reaktion gewonnenen 2-Alkylalkanolen mit 8 bis 32 C-Atomen verestert.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 537 901 (J.C. PURDUE) * Anspruch 1; Spalte 6, Zeile 73 - Spalte 7, Zeile 3 * --- | 6 | C 08 K 5/11 C 08 L 21/00 C 07 C 69/34 |
| X | WO-A-8 600 300 (HENKEL) * Anspruch 1; Seite 4, Zeilen 27-90 * --- | 6 | |
| X | DE-A-1 694 541 (KALLE) * Anspruch 1; Seite 4, Zeile 25 * --- | 6 | |
| A | EP-A-0 182 912 (FURUKAWA) * Anspruch 1 * ----- | 1 | |
| | | | **RECHERCHIERTE SACHGEBIETE (Int. Cl.5)** C 08 K C 08 L C 07 C |

Der vorliegende Recherchenbericht wurde für alle Patentansprüche erstellt

| Recherchenort | Abschlußdatum der Recherche | Prüfer |
|---|---|---|
| DEN HAAG | 17-01-1990 | VAN HUMBEECK F.W.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...........................................................................
& : Mitglied der gleichen Patentfamilie, übereinstimmendes Dokument